# EUROPEAN PATENT APPLICATION

(11) **EP 2 017 340 A1**
(43) Date of publication of application: **21.01.2009**
(21) Application number: 07013897.9
(22) Date of filing: 16.07.2007
(51) Int. Cl.: C12N 15/11, C12Q 1/30, C12N 9/08

(54) **Positive controls for expression modulating experiments**

(71) Applicant: Qiagen GmbH, 40724 Hilden (DE)
(72) Inventor: Dennig, Jörg, Dr., 40764 Langenfeld (DE); Narz, Frank, Dr., 58456 Witten (DE); Kaufmann, Brigitte, Dr., 42799 Leichlingen (DE)
(74) Representative: Schönbohm, Carla

(57) **Abstract**

The invention pertains to the use of an expression modulating compound such as an RNAi compound inhibiting the expression of catalase as positive control.

## Description

The present invention pertains to the use of certain expression modulating compounds as positive controls in assay systems as well as to the respective expression modulating compounds.

Many methods are known in the state of the art that target and/or influence gene expression. Earlier methods modified the DNA, e.g. by mutation or recombination. Such methods, however, altered the genetic identity of the organism.

Several new techniques were established in the last years that did not target the DNA but the RNA (mRNA) in order to modulate gene expression. These RNA-targeting techniques allow modulation of gene expression such that only minimal levels of functional mRNA remain, thereby allowing even the modulation of essential genes. These techniques allow the creation of specific phenotypes as well as the analysis of the gene function in different developmental stages.

One of the earlier methods of these RNA - targeting techniques was the antisense technology. Antisense polynucleotides are designed to specifically bind to RNA, resulting in the formation of RNA-DNA or RNA-RNA hybrids, with an arrest of reverse transcription or messenger RNA translation. Antisense polynucleotides based on a selected sequence can thus modulate and interfere with expression of the corresponding gene as the mRNA is targeted and e.g. translation is at least partially inhibited. Many forms of antisense have been developed and can be broadly categorized into enzyme-dependent antisense or steric blocking antisense. Enzyme-dependent antisense includes forms dependent on RNase H activity to degrade target mRNA, including single-stranded DNA, RNA, and phosphorothioate antisense.

Antisense polynucleotides are typically generated within the cell by expression from antisense constructs that contain the antisense strand as the transcribed strand. Antisense polynucleotides will bind and/or interfere with the translation of the corresponding mRNA. As such, antisense may be used for research purposes as well as therapeutically e.g. to inhibit the expression of oncogenes.

Antisense RNA or antisense oligodeoxynucleotides (antisense ODNs) can both be used and may also be prepared *in vitro* synthetically or by means of recombinant DNA techniques. Both methods are well within the reach of the person skilled in the art. ODNs are smaller than complete antisense RNAs and have therefore the advantage that they can more easily enter the target cell. In order to avoid their digestion by DNAse, ODNs and antisense RNAs may be chemically modified.

Trans-cleaving catalytic RNAs (ribozymes) are RNA molecules possessing endoribonuclease activity. Ribozymes are specifically designed for a particular target, and the target message must contain a specific nucleotide sequence. They are also established expression modulators. They are engineered to cleave any RNA species site-specifically in the background of cellular RNA. The cleavage event renders the mRNA unstable and prevents protein expression. Importantly, ribozymes can be used to inhibit expression of a gene of unknown function for the purpose of determining its function in an in vitro or in vivo context, by detecting the phenotypic effect.

A further mechanism for modulating expression on a post-transcriptional level is RNA interference (RNAi) which is a mechanism for RNA guided regulation of gene expression in which double-stranded ribonucleic acid molecules inhibit the expression of genes with complementary nucleotide sequences. Conserved in most eukaryotic organisms, the RNAi pathway is thought to have evolved as form of innate immunity against viruses and also plays a major role in regulating development and genome maintenance.

The RNAi pathway is initiated by the enzyme dicer, which cleaves double-stranded RNA (dsRNA) to short double-stranded fragments of usually approximately 20 to 25 base-pairs. One of the two strands of each fragment, known as the guide strand, is then incorporated into the RNA induced silencing complex (RISC) and base-pairs with complementary sequences. The most well-studied outcome of this recognition event is a form of post-transcriptional gene silencing. This occurs when the guide strand base-pairs with the messenger RNA (mRNA) molecule and induces degradation of the mRNA by argonaut, the catalytic component of the RISC complex. The short RNA fragments are known as small interfering RNA (siRNA) when they derive from exogenous sources and microRNA (miRNA) when they are produced from RNA coding genes in the cells' own genome.

The selective and robust effect of RNAi on gene expression makes it a valuable research tool, both in cell culture and in living organisms. Synthetic dsRNA introduced into cells can induce suppression of specific genes of interest. The effect of these genes on the phenotype of the cells can then be analyzed by studying the effect of the gene silencing. RNAi may also be used for large-scale screens that systematically shut down each gene in the cell, which can help identify the components necessary for a particular cellular process or an event such as for example, cell division.

Due to its advantages siRNA-mediated RNAi has become an indispensable tool in functional genomic research. Chemically synthesized siRNA reagents that target every gene in a human, mouse and rat genome are available for convenient delivery *in vitro.* Data acquired from RNAi experiments are used to support important conclusions about how genes function.

In addition to their role in the RNAi pathway, siRNAs also act in RNAi related pathways, for example as an antiviral mechanism or in shaping the chromatin structure of a genome; the complexity of these pathways is only now been elucidated.

For the above reasons, the RNA interference pathway is often exploited in experimental biology to study the function of genes in cell culture and *in vivo* in model organisms. Double-stranded RNA is synthesized with a sequence complementary to a gene of interest and introduced into the cell or organism, where it is recognized as exogenous genetic material and activates the RNAi pathway. Using this mechanism, researchers can cause a drastic decrease in the expression of the targeted gene. Studying the effects of this decrease can show the physiological role of the respective targeted gene product. Since RNAi may not totally abolish expression of the gene, this technique is sometimes referred to as a "knowdown" to distinguish it from "knockout" procedures in which expression of a gene is entirely eliminated.

Depending on the organism and the experimental system, the exogenous RNA may be a long strand designed to be cleaved by dicer or short RNAs designed to serve as siRNA substrates. In most mammalian cells, shorter RNAs are used because long double-stranded RNA molecules induce the mammalian interferon response, a form of innate immunity that reacts non-specifically to foreign genetic material.

They are usually introduced in the cells by appropriate tranfection methods.

Specialized laboratory techniques have also been developed to improve the utility of RNAi in mammalian systems by avoiding the direct introduction of siRNA, for example, by stable transfection with a plasmid encoding the appropriate sequence from which siRNA can be transcribed, or by more elaborate lentiviral vector systems allowing the inducible activation or deactivation of transcription, known as conditional RNAi.

In order to ensure that the conclusion drawn from any expression modulating experiment such as e.g. an antisense or a RNAi experiment are accurate, it is important to include proper controls in every expression modulating experiment. Such controls strengthen the drawn conclusions and ensure that the performed expression modulating experiments result in the expected silencing. Appropriate experimental controls are thus of utmost importance in order to maximize the value of the generated data.

Commonly, at least three types of control samples are run e.g. in every RNAi experiment: a positive control, a negative control and an untreated control. Positive controls monitor efficiency of e.g. siRNA delivery into cells (transfection efficiency) and negative controls distinguish sequence-specific silencing from non-specific effects. Untreated samples determine the baseline level of cell viability, the cell phenotype and target gene level.

In order to achieve successful gene silencing, efficient delivery of the expression modulating compound such as an siRNA into the cells of interest is important. E.g. siRNA delivery efficiency can vary between individual cell types used and the delivery method used. It has been shown, that basically all delivery methods bear the risk to produce a negative effect on cell viability. A well-characterized positive control for siRNA is thus an ideal reagent for establishing parameters that result in successful siRNA delivery, without affecting cell viability.

In RNAi experiments, as positive controls usually siRNA are used that target a housekeeping gene. Popular target genes are for example GAPDH, Lamine, MAPK1, Beta-Actine, p53, cyclophilin B and ciferase GL2.

The ideal positive control as presently aimed at in the state of the art thus targets a house keeping gene that this constitutively and abundantly expressed in a wide variety of cell types, the silencing of the chosen target gene as a positive control does not affect the cell viability, the silencing of the target gene does not affect the cell phenotype and the silencing of the target gene is convenient to assay.

Furthermore, the problem exists that siRNA positive controls used in the prior art often lead to cytotoxic effects. These effects are difficult to quantify and can often not be properly distinguished from unspecific cytotoxic effects of the actual siRNA transfection.

Furthermore, many positive controls demand elaborate and time-consuming testing methods. Many researchers are currently using quantitative RT-PCR or western blotting to assess the compound induced knockdowns. While accurate, these techniques can be very time-consuming and labor intensive. Another means to monitor expression modulating compound/siRNA delivery is the use of fluorescently labeled compounds/siRNAs. This method has the advantage of speed, but it can be unreliable since the compounds/siRNA can be trapped in endosomes or other subcellular compartments that keep the expression modulating compound such as a siRNA from directing their target mRNA.

There is thus a need for easy testing methods for the used controls. One assay known in the prior art aims to solve this problem by directing e.g. siRNAs against GAPDH (glyceraldehyde3-phospate-dehydrogenase), allowing to determine the silencing effect of the positive control siRNA enzymatically.

It is the object of the present invention to provide a positive control for use in expression modulating experiments, in particular RNAi experiments, which is effective and convenient to assay.

This object is solved by the use of at least one expression modulating compound directed against the expression of the enzyme catalase as a positive control in an expression modulating assay. The expression modulating compound which is used as a positive control according to the present invention inhibits expression of the enzyme catalase on the post-transcriptional level by targeting the catalase mRNA (catalase knockdown). By analyzing the efficiency of the catalase knockdown, efficiency of the delivery of the expression modulating compound into the cell can be controlled.

Catalase is a common enzyme found in living organisms. It functions include catalyzing the decomposition of hydrogen peroxide to water and oxygen. Catalase is a very active enzyme and has one of the highest turnover rates of all enzymes. Catalase is a tetramer of four polypeptide chains, each over 500 amino acids long. It contains four porphyrin heme groups which allow the enzyme to react with the hydrogen peroxid. The optimum pH for catalase is approximately neutral (pH 7,0), while the optimum temperature varies by species.

The use of an expression modulating compound such as an RNAi compound targeting the expression of catalase as a positive control has many important advantages. This, as catalase is ubiquitous and abundantly expressed in almost all cell types.

Furthermore, there are no other comparable enzyme functions in the cell that could lead to an undesired enzymatic background activity bearing the risk to falsify the results. Testing of the positive controls according to the present invention is also very convenient, as the effect on the catalase expression can be determined enzymatically by measuring the catalase activity in the cell. Measuring can even be performed photometrically. Determination of the influence on catalase expression can thus be done by easy and convenient assay methods, making time-consuming and elaborate methods such as the commonly used quantitative RT-PCR or western blotting obsolete. However, it is self-evident that these methods can be used in conjunction with the present invention.

Another important advantage of the positive control directed against catalase according to the present invention is that catalase is not an established housekeeping gene. The invention thus departs from common ways by not targeting an established housekeeping gene. The positive control according to the present invention is thus usable in conjunction with other established analysis systems, which aim at targeting housekeeping genes (for example for normalizing the obtained results).

Targeting the expression of catalase for use as a positive control has thus many important advantages.

As enzymatic assays are easy to handle, it is preferred that the effect of the expression modulating compound on the expression of catalase for measuring transfection efficiency is analyzed in an enzymatic assay. Therein, the cells comprising the expression modulating compound are lysed and the activity of catalase is then measured by using appropriate reagents. This is usually done photometrically, many commercially available test systems are known. Therefore, the analysis can be performed very easily using established systems.

Many compounds may be used in order to regulate the expression of catalase on a post-transcriptional level. Examples of appropriate expression modulators include but are not limited to small organic molecules, nucleic acids, peptides, cyclic peptides, antisense molecules, RNAi molecules, and ribozymes. Respective modulators are well-known to the skilled person and were also outlined above. Please refer to our above comments for details.

According to one embodiment, the expression modulating compound is a RNAi modulating compound, preferably an siRNA. As siRNA, said compound is a double-stranded molecule having 3' overhangs on each strand. The length of said siRNA is usually between 18 and 35 nt, preferably between 19 and 25 nt. The 3' overhangs on each end are preferably 2 nts long. In order to efficiently induce silencing, the siRNA used as RNAi modulating compound is substantially complementary to the catalase transcript for inhibiting the expression of said target transcript by RNA interference. Suitable siRNAs can be identified by using proper computational methods, applying certain design-algorithms. Several methods are known and can be used in conjunction with the present invention.

In order to obtain a siRNA of the above structure against the target transcript, the double-stranded molecule can be transfected directly into the cell. Alternatively, this structure may result by processing by dicer, an enzyme that converts either long dsRNAs or small hairpin RNAs (shRNAs) into siRNAs (see above). These precursors or the final siRNA molecules can be produced exogenously (artificially) and can then be introduced into the cells to be analyzed by various transfection methods, to analyze the specific knockdown of the catalase gene.

According to one embodiment, the expression modulating and in particular the RNAi modulating compound inhibiting the expression of catalase is expressed by a vector. This embodiment is advantageous, as e.g. transfection of an exogenous siRNA or antisense molecule can be sometimes problematic, since the gene knockdown effect is only transient, particularly in rapidly dividing cells. One way of overcoming this challenge is to modify the expression modulating compound such as a siRNA in such a way as to allow it to be expressed by an appropriate vector, for example a plasmid. For siRNA, this done by the introduction of a loop between the two strands, thus producing a single transcript, which can be then processed into a functional siRNA in the cell. Such transcription cassettes typically use an RNA polymerase 3 promoter (for example U6 or H1) which usually direct the transcription of small nuclear RNAs (shRNAs) (U6 is involved in gene's placing; H1 is the RNA subcomponent of human RNAse p). It is assumed that the resulting shRNA transcript from the vector is then processed by dicer, thereby producing the double-stranded siRNA molecules, having the characteristic 3' overhangs.

According to a preferred embodiment, the siRNA knocking down the expression of catalase is selected from the following sequences:
CAT-1:
   Target sequence: 5' CTG GAG TTA CAT TAA TGT TAA 3'
   Sense-strand: 5' r(GGA GUU ACA UUA AUG UUA A)dTdT 3'
   Antisense strand: 5' r(UUA ACA UUA AUG UAA CUC C)dAdG 3'
CAT-2:
   Target sequence: 5' ACC GTC ATG GCT TAA TGT TTA 3'
   Sense strand: 5' r(CGU CAU GGC UUA AUG UUU A)dTdT 3'
   Antisense strand: 5' r(UAA ACA UUA AGC CAU GAC G)dGdT 3'

The "r" indicates the ribonucleotide section, the "d" indicates desoxyribonucleotides. Therefore, the present invention also provides the above siRNAs which can be advantageously used as positive controls in RNAi experiments.

Furthermore, a method is provided for performing an expression modulating analysis/assay wherein at least one expression modulating compound inhibiting the expression of the enzyme catalase by targeting the RNA is used as a positive control to determine delivery efficiency.

Further embodiments/features of this method were already outlined above in conjunction with the description of the use of respective expression modulating compounds as positive controls. These features/embodiments equally apply to the provided method according to the present invention. This in particular pertains to the defined examples of expression modulating compounds and the embodiment, wherein the effect of said expression modulating compound such as an RNAi modulating compound on the expression of catalase is analyzed in an enzymatic assay, preferably photometrically.

As also outlined above, the RNAi modulating compound can be a siRNA. The siRNA is preferably substantially complementary to the catalase transcript for inhibiting the expression of said target transcript by RNA interference. Suitable examples of respective siRNAs and ways to generate them are described above and equally apply to the provided method.

According to a further embodiment of the method of the present invention, an internal control is used additionally. Said internal control is preferably a cellular component which can also be analyzed/determined via an enzymatic assay. Said internal control is preferably unaffected by the expression modulating compound(s) tested in the experiment. This embodiment allows the normalization without the need for elaborate and highly-sensitive analysis systems such as RT-PCR or systems based upon a Western-blot analysis.

According to one embodiment, a LDH assay is additionally performed. This embodiment has the advantage that if LDH (lactate dehydrogenase) is used as cellular component, also conclusions regarding potential cytotoxic effects may be drawn. LDM catalyses the conversion of lactate to pyruvate. The activity of the LDM enzyme may be determined photometrically in a NADH-coupled enzyme reaction. In case cytotoxic effects occur, LDH is found not only in the cells but also in the supernatant of the cell culture. The amount of LDH detected in the supernatant thus allows conclusions regarding the cytotoxic effects of the compound that is tested in the expression modulation experiment (e.g. a siRNA targeting a certain mRNA).

Also provided is an expression modulation analysis kit, comprising as one component an expression modulating compound, inhibiting expression of the enzyme catalase as a positive control. The kit may comprise further components, such as other expression modulating compounds such as e.g. siRNAs for knockdown of the desired target genes, negative controls, reagents, such as for example transfection reagents and buffers. In order to facilitate the enzymatic assay of the catalase activity in the respectively transfected cells, the kit may also comprise a lysis buffer and other reagents for analyzing the enzymatic activity of catalase.

The present invention shall be outlined in more detail by way of two examples. These examples are only for illustrating the beneficial effects of the present invention and are by no means limiting.

In order to silence the gene of the human catalase enzyme, two siRNAs were synthesized which target the catalase transcription as target sequence:
CAT-1:
   Target sequence: 5' CTG GAG TTA CAT TAA TGT TAA 3'
   Sense-strand: 5' r(GGA GUU ACA UUA AUG UUA A)dTdT 3'
   Antisense strand: 5' r(UUA ACA UUA AUG UAA CUC C)dAdG 3'
CAT-2:
   Target sequence: '5 ACC GTC ATG GCT TAA TGT TTA 3'
   Sense strand: 5' r(CGU CAU GGC UUA AUG UUU A)dTdT 3'
   Antisense strand: 5' r(UAA ACA UUA AGC CAU GAC G)dGdT 3'

For both of these siRNAs the functionality was proven by the following experiments:

### Experiment 1: functionality of the siRNAs

The aim of this functionality assay was to prove that both siRNAs lead to a silencing on an mRNA level. The cell type which was used for this experiment were HeLa cells. In order to transfect these cells QIAGEN's "HiPerFect Transfection Reagent" was used according to the QIAGEN standard protocol in 24-well plates.

After 48 hours of incubation, the silencing effect on catalase was analyzed. Therein, the catalase as well as the GAPDH-mRNA was analyzed by way of quantitative RT-PCR.

The analysis of the GAPDH expression was used as internal control. GAPDH expression is not influenced by the transfected siRNA. Deviations in the GAPDH measurement reflect thus experimental fluctuations/deviations that may be deducted/excluded from the obtained catalase-measurement. Alternatives for GAPDH basically are any genes that are not regulated by the transfected expression modulating compounds (test compounds as well as controls). Suitable are particularly house-keeping genes, as their expression is rather stable even when manipulations such as transfections occur.

As controls untransfected cells and a transfection with a negative control siRNA (Allstar Negative Control - QIAGEN) were used. The relative catalase mRNA amount in the cell was determined by quantitative RT-PCR and was normalized compared to the GAPDH amount. Two different concentrations of siRNA were used, 50 nmol and 5 nmol.

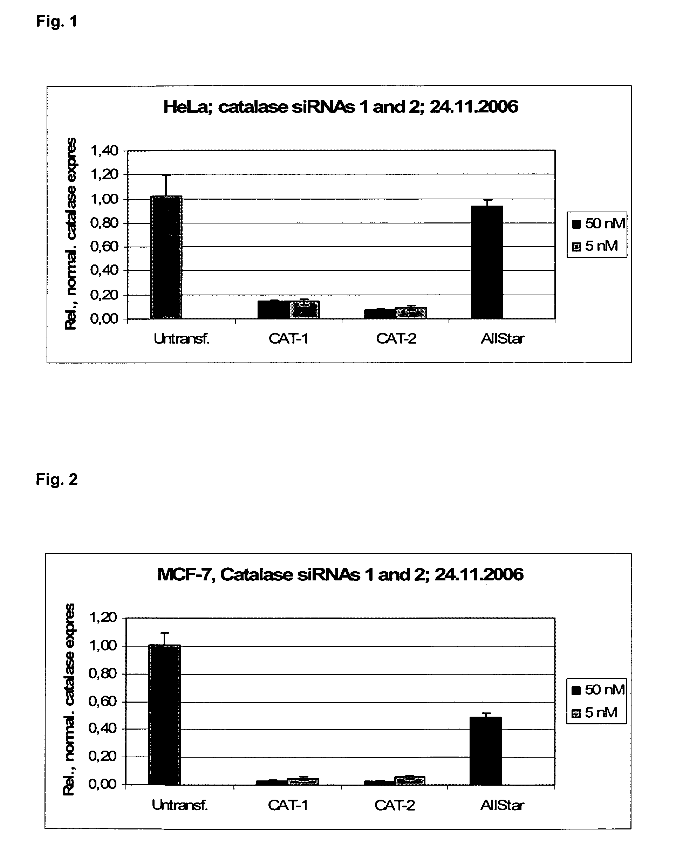

The results are demonstrated in figure 1. As can be seen, both siRNAs are functionally active in HeLa cells. For both concentrations and for both siRNAs (CAT-1 and CAT-2) less than 14 % of the catalase mRNA starting amount was found after siRNA transfection in the HeLa cells. This demonstrates that the siRNAs are functionally active.

### Example 2: functionality of the siRNAs

The second experiment also aimed to prove in a different cell type that both siRNAs lead to a silencing of the catalase expression on a mRNA level. In this experiment, MCF-7 cells were used. Again, as a transfection method the "HiPerFect Transfection Reagent" was used, according to the handbook's so called Traditional Transfection Protocol (QIAGEN). The cells were transfected in 24-well-plates. The results of the gene silencing was also analyzed after 48 hours. As controls untransfected cells and a transfection of a negative control-siRNA (Allstar Negative Control - QIAGEN) was used. As in experiment 1, the relative catalase mRNA amount was determined by quantitative RT-PCR and normalized compared to the GAPDH amount. As in experiment 1, siRNA concentrations of 50 nmol and 5 nmol were used. The results are shown in figure 2.

In MCF-7 cells, less than approximately 6 % of the catalase mRNA starting amount was found. This demonstrates the powerful silencing effect of the used siRNAs.

### Experiment 3: detection of the gene silencing effect in an enzymatic assay

As outlined above, it is convenient to use enzymatic methods in order to determine the gene silencing effect of the positive control knocking down catalase. Experiment 3 aimed to prove, that the gene silencing effect can be determined by way of an enzymatic assay. HeLa cells were used in this experiment. As transfection agent, "HiPerFect Transfection Reagent" was used according to the handbook's so called Traditional Transfection Protocol (QIAGEN). The experiments were done in 96-well plates. The analysis was performed after 48, 72 and 96 hours. For the analysis, the catalase assay kit, Cayman chemical (catalogue number 707002) was used.

The transfected cells were lysed 48, 72 and 96 hours after transfection and analyzed. For lysis, a usual lysis buffer was used (10 mM Tris; pH 7,4; 1 mM EDTA, 100 mM NaCl; 5 mM MgCl₂; 1 % NP 40).

The catalase activity was already below 40 % of initial activity after 48 hours. This is an important finding as it can be excluded, that the gene silencing is only detectable after a few days on a protein level (for example due to a high protein stability). The finding, that an effect on the protein level is found early, simplifies the practical use of the RNAi modulating compound according to the present invention targeting the catalase gene.

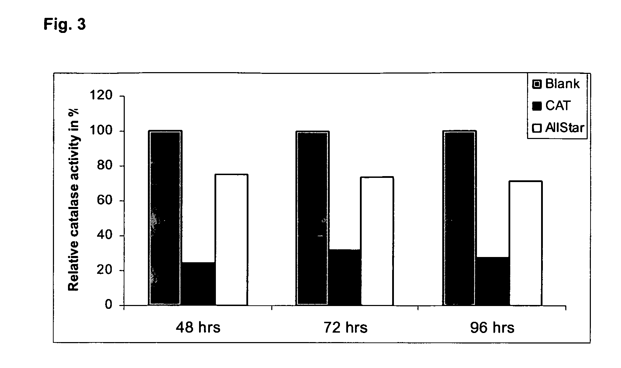

The results of experiment 3 are shown in figure 3.

## Claims

1. Use of at least one expression modulating compound silencing the expression of the enzyme catalase as a positive control in an expression modulating assay.

2. Use according to claim 1, wherein the effect of said expression modulating compound on the expression of catalase is analyzed in an enzymatic assay.

3. Use according to claim 1 or 2, **characterized in that** the expression modulating compound is an RNAi modulating compound such as an siRNA or an shRNA, an antisense molecule or a ribozyme.

4. Use according to claim 3, wherein the RNAi modulating compound is an siRNA double-stranded molecule of preferably 18 to 30 nt, having 3' overhangs.

5. Use according to at least one of the preceding claims 1 to 4, wherein said expression modulating compound is expressed by a vector.

6. Use according to at least one of the preceding claims 3 to 5, wherein said siRNA is preferably selected from
CAT-1:
Sense-strand: 5' r(GGA GUU ACA UUA AUG UUA A)dTdT 3'
Antisense strand: 5' r(UUA ACA UUA AUG UAA CUC C)dAdG 3'
or
CAT-2:
Sense strand: 5' r(CGU CAU GGC UUA AUG UUU A)dTdT 3'
Antisense strand: 5'r(UAA ACA UUA AGC CAU GAC G)dGdT 3'

7. siRNA selected from
CAT-1:
Sense-strand: 5' r(GGA GUU ACA UUA AUG UUA A)dTdT 3'
Antisense strand: 5' r(UUA ACA UUA AUG UAA CUC C)dAdG 3'
or
CAT-2:
Sense strand: 5' r(CGU CAU GGC UUA AUG UUU A)dTdT 3'
Antisense strand: 5' r(UAA ACA UUA AGC CAU GAC G)dGdT 3'

8. A method for performing an expression modulating analysis or assay, wherein at least one expression modulating compound inhibiting the expression of the enzyme catalase by targeting the catalase mRNA is used as a positive control.

9. Method according to claim 8, wherein the effect of said expression modulating compound on the expression of catalase is analyzed in an enzymatic assay, by RT-PCR or by western blot analysis.

10. Method according to claim 9, wherein the catalase activity is determined photometrically.

11. Method according to at least one of the claims 8 to 10, wherein the expression modulating compound is an RNAi modulating compound such as an siRNA or an shRNA, an antisense molecule or a ribozyme.

12. Method according to claim 11, wherein said RNAi modulating compound is an siRNA or an shRNA, which is substantially complementary to the catalase mRNA for inhibiting the expression of said target mRNA by RNA interference.

13. Method according to claim 11 or 12, wherein the siRNA is used in form of a double-stranded molecule of preferably 19 to 30 nts, having 3' overhangs.

14. Method according to at least one of the preceding claims 10 to 13, wherein the siRNA used as a positive control is preferably selected from
CAT-1:
Sense-strand: 5' r(GGA GUU ACA UUA AUG UUA A)dTdT 3'
Antisense strand: 5' r(UUA ACA UUA AUG UAA CUC C)dAdG 3'
or
CAT-2:
Sense strand: 5' r(CGU CAU GGC UUA AUG UUU A)dTdT 3'
Antisense strand: 5' r(UAA ACA UUA AGC CAU GAC G)dGdT 3'

15. Method according to at least one of the preceding claims 8 to 14, wherein at least one expression modulating compound is used as test compound which inhibits the expression of a target different from catalase.

16. Method according to claim 15, wherein said expression modulating compound is an RNAi modulating compound such as an siRNA or an shRNA, an antisense molecule or a ribozyme.

17. Expression modulation kit, in particular for RNAi experiments, comprising an expression modulating compound inhibiting expression of the enzyme catalase as a positive control.

18. Kit according to claim 17, comprising a lysis buffer and reagents for analyzing the enzymatic activity of catalase.
